(19) Europäisches Patentamt
European Patent Office
Office européen des brevets

(11) Veröffentlichungsnummer: **0 293 913 B1**

# (12) EUROPÄISCHE PATENTSCHRIFT

(45) Veröffentlichungstag der Patentschrift: **11.09.91 Patentblatt 91/37**

(51) Int. Cl.$^5$: **C07C 303/06**

(21) Anmeldenummer: **88108913.0**

(22) Anmeldetag: **03.06.88**

## (54) Verfahren zur Herstellung von gegebenenfalls substituierten Ethansulfonverbindungen.

(30) Priorität: **04.06.87 DE 3718774**

(43) Veröffentlichungstag der Anmeldung: **07.12.88 Patentblatt 88/49**

(45) Bekanntmachung des Hinweises auf die Patenterteilung: **11.09.91 Patentblatt 91/37**

(84) Benannte Vertragsstaaten: **AT BE CH DE ES FR GB GR IT LI LU NL SE**

(56) Entgegenhaltungen:
**DE-A- 1 518 400**
**DE-A- 2 249 360**
**US-A- 3 200 127**
**"Ullmanns Encyklopädie der technischen Chemie", Band 22, 1982, Seiten 480-482**

(73) Patentinhaber: **Raschig AG**
**Mundenheimer Strasse 100**
**W-6700 Ludwigshafen/Rhein (DE)**

(72) Erfinder: **Clauss, Wolfgang**
**Waldstrasse 21**
**W-6730 Neustadt (DE)**
Erfinder: **Fell, Bernhard**
**Im Mittelfeld 6**
**W-5100 Aachen (DE)**
Erfinder: **Hendricks, Günter**
**Worringerweg 1**
**W-5100 Aachen (DE)**
Erfinder: **Kurze, Werner**
**Wiesenstrasse 17**
**W-6708 Neuhofen (DE)**
Erfinder: **Lämmerzahl, Frank**
**Am Markt 21**
**W-6900 Heidelberg (DE)**
Erfinder: **Wassenberg, Willy**
**Speyrer Wingert 47**
**W-6708 Neuhofen (DE)**

(74) Vertreter: **Berendt, Thomas, Dr.rer.nat. Dipl.-Chem. et al**
**Patentanwälte Dr.rer.nat. Dipl.-Chem. Th. Berendt Dr.Ing. Hans Leyh Innere Wiener Strasse 20/III**
**W-8000 München 80 (DE)**

EP 0 293 913 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von gegebenenfalls substituierten Ethansulfonverbindungen.

Verbindungen der Ethansulfonsäure entstehen in hohen Ausbeuten, wenn man das Primärprodukt der Sulfonierung von Olefinen mit Schwefeltrioxid oder deren Komplexe, z.B. mit Dioxan, mit sauerstoff- oder stickstoffhaltigen Verbindungen, z.B. mit Alkoholen, Aminen, Pyridinen umsetzt. Als mögliche Zwischenprodukte der Sulfonierung werden nicht in Substanz faßbare 1,2-Sultone (= 1,2-Oxathietan-2,2-dioxidverbindungen) angenommen, die zu stabileren 1,3-Sultonen und 1,4-Sultonen und insbesondere zu Alkensulfonsäuren isomerisieren. Gleichzeitig können auch noch andere Nebenprodukte, wie z.B. β-Disultone oder homologe Carbylsulfate gebildet werden. (Ullmanns Encyklopädie der technishen Chemie Bd. 22 (1982), Seiten 480 bis 482). Durch Hydrolyse bilden sich Hydroxyalkansulfonsäuren, durch Umsetzen mit Ammoniak oder primären Aminen, wie Aminosäuren, in relativ geringer Ausbeute die entsprechenden Aminoverbindungen in 2-Stellung des Alkanrestes. (C.A.Bd. 62 (1965) Spalte 14502 f bis 14503 a).

Es wurde gefunden, dass bei einer bestimmten Verfahrendurchführung im Fallfilmreaktor oder Dünnschichtreaktor eine hohe Ausbeute insbesondere langkettiger 2-Alkyl-2-alkoxy-ethan-sulfonsäuren oder 2-Alkyl-2-amino-ethansulfonsäuren, die als oberflächenaktive Mittel (Tenside) brauchbar sind, erhalten werden.

Verbindungen, die den genannten Ethansulfonsäuren sehr ähnlich sind, lassen sich durch Reaktion von Hydroxyl- und Aminoverbindungen mit 1,3-Propansulton herstellen.

1,3-Propansulton besitzt gegenüber den 1,2-Sultonen eine erheblich geringere Reaktivität, so daß bei den Umsetzungen mit den genannten Hydroxyl- und Aminoverbindungen höhere Reaktionstemperaturen und längere Reaktionszeiten in Kauf genommen werden müssen. Ebenfalls verhindert die geringere Reaktivität des 1,3-Propansultons in vielen Fällen einen vollständigen Umsatz des 1,3-Sultons, so dass die Endprodukte durch toxische 1,3-Propansultonspuren verunreinigt werden können.

Für die Erzielung einer hohen Ausbeute an 1,2-Sulton ist die Sulfonierung mit komplexiertem $SO_3$ notwendig. Die Sulfonierung mit freiem $SO_3$, z.B. in einem Fallfilmreaktor, führt auch bei sehr tiefen Temperaturen von z.B. −10°C nur zu geringen Ausbeuten an 1,2-Sultonen und damit auch an den gewünschten Ethansulfonverbindungen. Da der $SO_3$-Dioxan-Komplex in allen für die Sulfonierung geeigneten Lösungsmitteln, z.B. Dichlorethan, Methylenchlorid, Alkanen unlöslich ist, kann die Olefinsulfonierung zu 1,2-Sultonen praktisch nicht in den für die Sulfonierung besonders gut geeigneten, kontinuierlich arbeitenden Fallfilmreaktoren ausgeführt werden.

Es wurde nun gefunden, dass diese Nachteile vermieden werden können, wenn man erfindungsgemäß unter bestimmten, an sich bekannten Bedingungen die Olefinsulfonierung durchführt und unmittelbar darauf die Weiterreaktion mit bestimmten Hydroxyl-bzw. sekundären und tertiären Aminoverbindungen durchführt, wobei eine ganze Palette wertvoller oberflächenaktiver Substanzen erhalten werden kann.

Das erfindungsgemäße Verfahren zur Herstellung von gegebenenfalls substituierten Ethansulfonverbindungen der allgemeinen Formel

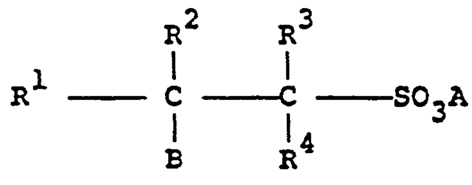 (1)

in der $R^1$ einer $C_1$-$C_{20}$-Alkylgruppe, $R^2$, $R^3$ and $R^4$ jeweils Wasserstoff oder eine $C_1$-$C_{20}$-Alkylgruppe bedeuten, A für Wasserstoff, Alkalimetall oder Ammonium oder eine negative Ladung und B für eine Gruppe $R^5O$, in der $R^5$ eine $C_1$-$C_{20}$-Alkyl-, oder -Alkylcarbonylgruppe, $C_2$-$C_{20}$-Alkenylcarbonylgruppe, $C_4$-$C_{20}$-Alkadienylgruppe oder Alkadienylcarbonylgruppe, Arylgruppe, Alkarylgruppe mit 1 bis 20 C-Atomen im Alkylrest, oder eine Carbonsäureamidoalkylgruppe mit 1 bis 20 C-Atomen in der Alkylgruppe steht, oder B für eine Gruppe $—O(CH_2CH_2O)_nR^5$ oder

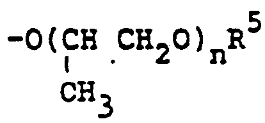 oder 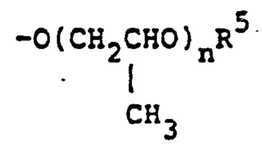

mit n = 1 bis 50 steht oder eine Gruppe $R^6R^7R^8N$ bedeuten, in der $R^6$, $R^7$ und $R^8$ je eine $C_1$-$C_{20}$-Alkylgruppe, oder -Alkylcarbonylgruppe oder eine Arylgruppe oder Alkarylgruppe mit 1 bis 20 C-Atomen im Alkylrest bedeutet, oder eine der Gruppen $R^6$ bis $R^8$ Wasserstoff ist, oder 2 der 3 Gruppen $R^6$ bis $R^8$ zusammen mit Stickstoff einen gegebenenfalls durch eine, zwei oder drei $C_1$-$C_5$-Alkylgruppen substituierten heterocyclischen Ring mit 5 oder 6 Gliedern aus Kohlenstoff- sowie gegebenenfalls ein oder zwei Sauerstoff- und|oder Stickstoffatomen bilden, ist dadurch gekennzeichnet, dass man ein Olefin der allgemeinen Formel

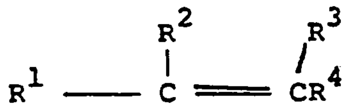

(11)

in der $R^1$, $R^2$, $R^3$ und $R^4$ die genannte Bedeutung haben, in einem inerten Lösungsmittel in Gegenwart von Dioxan bei einer Temperatur von +20°C bis –78°C mit Schwefeltrioxid, verdünnt mit Stickstoff oder einem gegen $SO_3$ inerten Gas, in einem Fallfilmreaktor sulfoniert und das erhaltene 1,2-Sulton der allgemeinen Formel

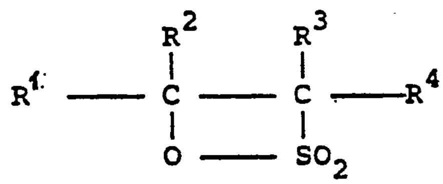

(III)

in der $R^1$ bis $R^4$ die genannte Bedeutung haben, ohne Isolierung entweder

a) durch Umsetzen, mit einer Verbindung der allgemeinen Formal $R^5OH$ oder deren Alkalisalz, worin $R^5$ die genannte Bedeutung hat, zur entsprechenden Sulfonsäure der allgemeinen Formel

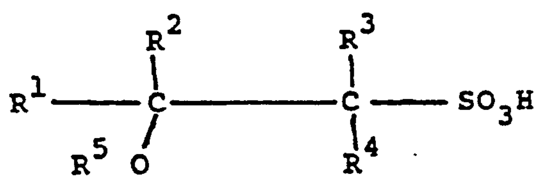

(IV)

umsetzt und diese gegebenenfalls nach Umsetzen mit der entsprechenden Base in das Alkali- oder Ammoniumsalz überführt oder

b) durch Umsetzen mit einem sekundären oder tertiären Amin der allgemeienen Formel $R^6R^7R^8N$, worin $R^6$, $R^7$ und $R^8$ die genannte Bedeutung haben, in die entsprechende Betainverbindung der allgemeinen Formel

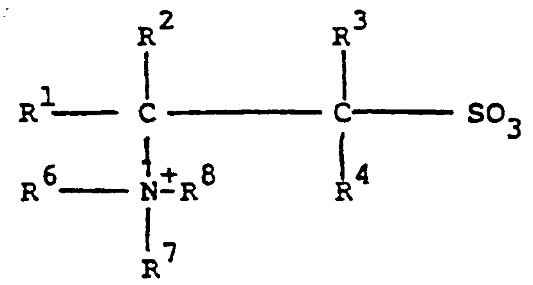

(V)

überführt.

Bevorzugte Alkylgruppen $R^1$ bis $R^8$ sind einmal $C_1$ bis $C_5$-Niederalkylgruppen und andererseits $C_{14}$ bis $C_{18}$-Alkylgruppen, wobei die Alkylgruppen gradkettig oder verzweigt sein können. Vorzugsweise handelt es sich um Verzweigungen, die Methyl-, Ethyl- oder Propylgruppen darstellen, welche an eine gradkettige höhere Alkylgruppen gebunden sind. Dies gilt auch für die Alkenylcarbonyl und Alkadienylcarbonylgruppen, sowie den Alkylrest der Alkarylgruppe $R^5$. Auch bei diesen Resten $R^5$ sind einerseits Niederalkylgruppen bis zu 5 Kohlenstoffatomen und andererseits $C_{14}$ bis $C_{18}$-Alkylgruppen bevorzugt. Bei den Arylgruppen $R^5$ bis $R^8$ sind Phenylgruppen bevorzugt, was auch für den Arylrest der Alkarylgruppen gilt. Bei den Alkenylcarbonylgruppen $R^5$ sind die von Acrylsäure und Methacrylsäure abgeleiteten Gruppen besonders bevorzugt.

Sofern zwei der Gruppen $R^6$ bis $R^8$ einen heterocyclischen Ring zuammen mit den Stickstoffatmen bilden, sind Pyridin-, Piperidin-, Morpholin — und Lutidinreste bevorzugt. Die dritte Gruppe ist in diesem Fall entweder Wasserstoff oder eine N=C — Doppelbindung oder eine Alkylgruppe, vorzugsweise mit 1 bis 5 C-Atomen.

Die in erster Stufe bei der Olefinsulfonierung erfindungsgemäß in hoher Ausbeute erhaltenen 1,2-Ethansultonverbindungen setzen sich bei der Weiterreaktion gemäß a) oder b) vollständig um, sodass mineral salzfreie Reaktionsprodukte erhalten werden.

Die Sulfonierung des Olefins mit $SO_3$ zum Primärprodukt der Sulfonierungsreaktion erfolgt unter Einhaltung möglichst milder Reaktionsbedinungen, d.H. bei den genannten tiefen Temperaturen und gegebenenfalls unter Verwendung inerter Lösungsmittel. Letzteres ist notwendig bei Einsatz von niedermolekularen, unter Normalbedingungen gasförmigen Olefinen, wie z.B. Propen, -Buten-2 oder Isobuten. Als inerte Lösungsmittel können z.B. Alkane oder auch chlorierte Kohlenwasserstoffe, wie Dichlormethan oder Dichlorethan verwendet werden. $SO_3$ wird gasförmig mit Luft, Stickstoff oder anderen inerten Gasen verdünnt. bis zu einer Konzentration von 1-20, vorzugsweise 3 bis 8 Vol.-%. Die Sulfonierung selbst kann vorteilhafterweise kontinuierlich in Fallfilmreaktoren bekannter Bauart (Dünnschichtreaktor), TO-Reaktor, vgl. Ullman a.a.O.) ausgeführt werden. Das Reaktorenendprodukt wird umittelbar und ohne Verweilzeit der Sulfoalkylierungsreaktion mit der gewünschten Verbindung a) oder b) zugeführt. Das Molverhältnis Olefin : $SO_3$ beträgt bei der Sulfonierung 1 : 1 bis 1 : 5 vorzugsweise 1 : 1.

Geringe Anteil an Olefinsulfonsäuren, die auch im Primärprodukt der Olefinsulfonierung bereits vorliegen, können nach der Sulfoalkylierungsreaktion durch übliche Trennoperation wie Umkristallisation, adsorptive Filtrations- oder andere bekannte Verfahren abgetrennt werden. Olefine, die bei der Sulfonierung mit $SO_3$ keine 1,3-Sultone bilden können, wie z.B. Propen, n-Buten-2 oder Isobuten sind besonders geeignete Ausgangsstoffe für diese Sulfoalkylierungsreaktionen.

Ein besonderer Vorteil der nach dem hier beschriebenen erfindungsgemäßen Sulfoalkylierungsverfahren hergestellten Produkte sind die — verglichen mit anderen Herstellungsverfahren — niedrigen Herstellungskosten. Die Produkte fallen z.B. mineralsalzfrei und elektrolytfrei an, wodurch ihre Reindarstellung und Anwendung bedeutend erleichtert wird.

Ein wichtiges Anwendungsbeispiel für die durch Sulfoalkylierung von Hydroxylgruppen-haltigen Substraten zugänglichen Ethersulfonate, ist die tertiäre Erdölförderung, bei der sie den hier ebenfalls eingesetzten Ethercarboxylaten wegen ihrer verminderten Retention am Speichergestein überlegen sind (Kosswig, Chiuz 18 (1984) 87).

Die große Variationbreite der Verbindungen gemäß der Erfindung durch gezielte Wahl der Reste $R^1$ bis $R^8$, A und B erlaubt eine optimale Anpassung der grenzflächenaktiven und anwendungstechnischen Eigenschaften der Produkte für spezielle Leistungsanforderungen z.B. bei Wasch- und Reinigungsmitteln, Kosmetika, Toilettenerzeugnissen und sonstigen sog. personal care products, Textilhilfsmitteln, Emulgatoren für die vershiedensten Zwecke, Reinigsmittel für die Metallindustrie, bei Tensiden für die Erdölaufarbeitung (Desmulgatoren), bei Tensiden für die Chemie- und Papierindustrie oder als Sammler und Schäumer in der Flotationstechnik (Surfactants in Consumer Products, Springer-Verlag Berlin-Heidelberg, New York 1987).

Auch die amphoteren Sulfobetaine, die sich von tertiären Aminen bzw. Pyridin oder Pyridinabkömmlingen herleiten, können durch gezielte Variation der Reste $R^1$ bis $R^8$ gewünschten anwendungstechnischen Eigenschaften optimal angepaßt werden. Sie werden als Tenside mit gute Hartwasserbeständigkeit, Unempfindlichkeit gegenüber Elektrolyten, (wichtig u.a. für den Einsatz bei der tertiären Erdölförderung) guter Haut- und schleimhautverträglichkeit, Kompatibilität mit anderen ionischen und nichtionischen Tensiden sowie antistatischen und textilweichmachenden Eigenschaften eingesetzt.

Der Einsatz dieser interessanten Tensidklasse in Haushaltsprodukten ebenso wie in Erzeugnissen für Chemie und Technik wird infolge verminderter Gestehungskosten durch das erfindungsgemäße Verfahren begünstigt.

In der beigefügten Zeichnung ist ein Fallfilmreaktor dargestellt, wie er für die in den folgenden Beispielen aufgeführten Versuche verwendet wurde.

Der Fallfilmreaktor besteht aus einem gekühlten Glasrohr (1 = ca. 60 cm, d = ca. 3 cm), an dessen senkrechten Innenwänden das Olefin-Dioxan-Lösungsmittel-Gemisch herunterläuft. Im Gleichstrom wird das Gas durch eine Fritte 2 oder durch ein perforiertes Glasrohr mit dem von Olefingefäß 7 mit dem Lösungsmittelgefäß 6 in die Pumpe 8 eingepumpten Flüssigkeitsfilm im Reaktor 1 in Kontakt gebracht. Als Gas wird das Luft-$SO_3$-Gemisch aus der kontinuierlichen Anlage verwendet. Als Lösungsmittel wird vorzugsweise Dichlorethan eingesetzt. Das Verhältnis von $SO_3$ zu Olefin in einer Zeiteinheit ist z.B. äquimolar. Unterhalb des Fallfilmreaktors befindet sich ein Gasableitungsrohr und Tropftrichter 5 als auffanggefäß für das flüssige Produktgemisch. Dieses wird zuerst im Abtrenngefäß 9 aufgefangen und dann das Abgas durch eine Waschvorrichtung 10, z.B. eine Waschflasche mit Schwefelsäure gelenkt.

Die Erfindung wird durch folgende Beispiele näher erläutert. Hierin ist RT = Raumtemperature = ca. 20°C.

## Beispiel 1

### Sulfonierung von n-Penten-(1) mit $SO_3$-Dioxan-

Das n-Penten-(1) wird mit der äquimolaren Menge an 1,4-Dioxan versetzt und soweit mit $C_2Cl_2H_4$ verdünnt, daß ein gleichmäßiger Fallfilm im Reaktor gebildet wird.

Die organische Phase wird mittels einer Pumpe in den Reaktor gefördert. Die Pumpeneinstellung wird so gewählt, daß in der Zeiteinheit äquimolare Mengen $SO_3$ und n-Penten –1 in den Reaktor gelangen. Der Kühlmantel wird mittels ein Kryostaten mit Kühlwasser beschickt.

Zum Abfangen der 1,2-Sultone wird die Reaktionslösung zu den jeweils angegebenen Reaktionszeiten mit überschüssigem Methanol gequenscht, wobei die entsprechende 2-Methoxyalkansulfonsäure entstehen. Die 1,3- und 1,4-Sultone können ohne Derivatisierung gaschromatographisch nachgewiesen werden.

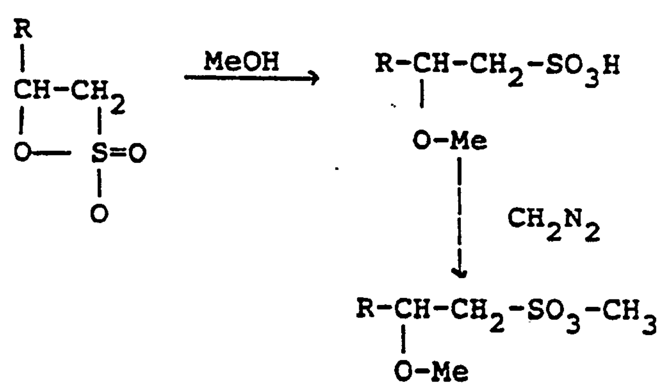


Zur Aufarbeitung zur gaschromatographischen Untersuchung wurde von der gequenschten, über Nacht bei Raumtemperatur gerührten Probe der Reaktionslösung eine kleine Menge (1-2ml) in einen Porzellantiegel gegeben und so lange eine etherische Diazomethanlösung zugetropft, bis das Diazomethan im Überschuß vorliegt (gelbe Lösung und keine Gasentwicklung mehr). Anschließend werden die Lösungsmittel durch einen Argonstrom abgeraucht und der meist zweiphasige Rückstand in 4-5 Tropfen Methanol homogen gelöst. Auf diese Weise werden all freien Sulfonsäuren in die entsprechenden Sulfonsäuremethylester überführt, die ohne Probleme gaschromatographisch identifiziert werden können.

Bei Verwendung von 8,07 Vol.-% $SO_3$ im Gasgemisch und einem Umsatz von 63,6 $cm^3$ Gas pro Stunde mit 0,09 Mol Penten-1/h in Gegenwart von 0,38 Mol 1,4-Dioxan (Verhältnis $SO_3$ : Dioxan = 1 : 4) betrug die Ausbeute an entsprechendem Ethansulfonsäuremethylester 93%. Als Nebenprodukte wurden 3% des 1,3-Sultons und 1,2% des 1,4-Sultons und 2,8% Dimethylsulfat erhalten.

Beispiel 2

Darstellung von 1-(1-n-Propyl-2-sulfoethyl)-pyridinium-betain im Fallfilmreaktor :

Die Sulfonierungsanlage gemäß Zeichnung wird mit folgenden Reaktionsparametern eingestellt :

| | |
|---|---|
| $SO_3$ : | 4 Vol.-%, 0,2 Mol/h |
| Penten 1 : | 0,2 Mol/h in Dichlorethan |
| Dioxan : | 0,4 Mol/h |
| Temperatur : | 19°C |

Die Versuchsdauer beträgt 2 Stunden. Der untere Teil der Anlage wird um einen Dreihalskolben mit mechanischem Rührer und Kühler mit Trockenrohr erweitert. Im Kolben befand sich, bezogen auf die Menge an durchgesetztem $SO_3$, die vierfache Menge an Pyridin (130 g) in 100 ml $C_2Cl_2H_4$. Der Kolben wurde durch ein Eisabd auf 0°C gekühlt.

Während des Zutropfens der Reaktionslösung fiel das Pyridiniumsulfobetain aus. Nach Beendigung der Reaktion wurde das Rohprodukt abfiltriert, aus EtOH umkristallisiert und getrocknet.

Ausbeute : 50 g = 53%, bezogen auf die eingesetzte Menge an $SO_3$.

Fp. 243-247°C.

Laut IR, 1H-NMR handelt es sich um folgende Verbindung

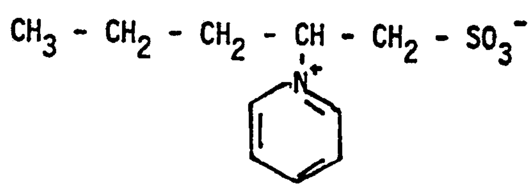

Elementaranalyse :

| | |
|---|---|
| Theorie : | C = 52,39, H = 6,59, N = 6,15, S = 13,98 |
| gefunden : | C = 52,50, H = 6,70, N = 6,20, S = 14,00 |

Beispiel 3

Darstellung von 1-(1-n-Propyl-2-sulfoethyl)-anilinium-betain

Die Versuchsbedingungen und die Aufarbeitung entsprechen Beispiel 2.

Ausbeute : 45 g = 46%, bezogen auf die eingesetzte Menge $SO_3$

Fp. : 214-217°C

Laut I.R.-Spektren und $^1$H-NMR handelt es sich um folgende Verbindung :

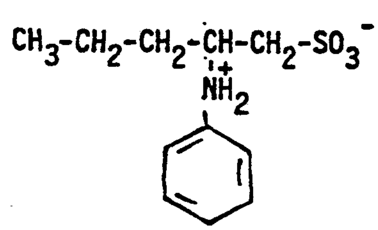

Analyse :

| | |
|---|---|
| berechnet : | C = 54,29, H = 7,04, N = 5,76, S = 13,18 |
| gefunden : | C = 54,00, H = 7,10, N = 5,80, S = 13,20 |

Beispiel 4 :

Darstellung von 1-(1-n-Propyl-2-sulfoethyl)-isopropylammonin-betain

Darstellungsweise gemäß Beispiel 2 :
Ausbeute : 33 g-40%, bezogen auf eingesetztes $SO_3$
Fp. : 225-229°C
Gemäß IR-Spektren und $^1$H-NMR handelt es sich um folgende Verbindung

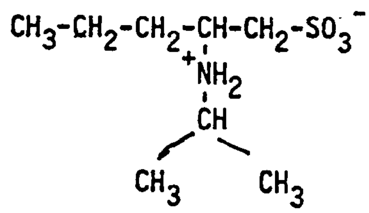

Analyse :

| | |
|---|---|
| berechnet : | C = 45,91, H = 9,15, N = 6,69, S = 15,32 |
| gefunden : | C = 45,70, H = 9,30, N = 6,70, S = 15,50 |

Beispiel 5

Darstellung von (1-Methyl-2-sulfoethyl)-acrylsäureester, K-Salz :

Die Reaktionsparameter entsprachen Beispiel 2.
Die Versuchsdauer betrug 2 h.
Allerdings wurde im Gegensatz zu der Vorgehensweise bei den Beispielen 2 bis 4 kein vierfacher Überschuß an 1,2-Acrylat verwendet, sondern eine, bezogen auf die eingesetzte Menge an $SO_3$, äquimolare Menge, suspendiert in Aceton (250 ml).
Nach Beendigung der Reaktion wird das Produkt abfiltriert und mehrmals mit Aceton gewaschen und anschließend getrocknet.
Ausbeute : 53,2 g = 52%, bezogen auf eingesetztes $SO_3$
Laut IR-Spektren und $^1$H-NMR handelt es sich um folgende Verbindung

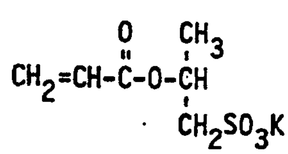

Beispiel 6

Darstellung von 2-(N-Morpholino)pentylsulfonsäure :

Es wurde gemäß Beispiel 2 gearbeitet.
Im Gegensatz zu den Versuchen mit Pyridin, Anilin und Isopropylamin, wo kristalline Produkte anfallen, bildet sich bei der Reaktion mit Morpholin kein Niederschlag. Nach abziehen der organischen Phase bleibt ein hochviskoses, harzartiges Produkt zurück, welches sich in polaren Lösungsmitteln, wie Wasser und Methanol, sehr leicht löst.

**Patentansprüche**

1. Verfahren zur Herstellung von gegebenenfalls substituierten Ethansulfonverbindungen der allgemeinen Formel :

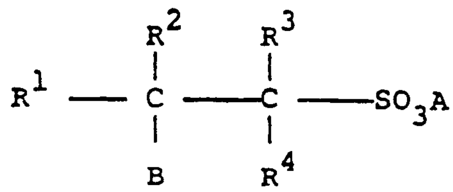

(1)

in der $R^1$ eine $C_1$-$C_{20}$-Alkylgruppe, $R^2$, $R^3$ und $R^4$ jeweils Wasserstoff oder eine $C_1$-$C_{20}$-Alkylgruppe bedeuten, A für Wasserstoff, Alkalimetall oder Ammonium oder eine negative Ladung und B für eine Gruppe $R^5O$, in der $R^5$ eine $C_1$-$C_{20}$-Alkyl-, oder -Alkylcarbonylgruppe, $C_2$-$C_{20}$-Alkenylcarbonylgruppe, $C_4$-$C_{20}$-Alkadiengruppe oder -Alkadienylcarbonylgruppe, Arylgruppe, Alkarylgruppe mit 1 bis 20 C-Atomen im alkylrest, oder eine Carbonsäureamidoalkylgruppe mit 1 bis 20 C-Atomen in der Alkylgruppe ist, oder B für eine Gruppe $—O(CH_2CH_2O)_nR^5$ oder

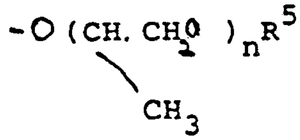

oder

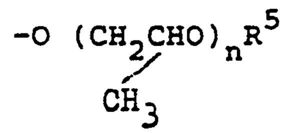

mit n = 1 bis 50 steht oder eine Gruppe $R^6R^7R^8N$ bedeutet, in der $R^6$, $R^7$ und $R^8$ je eine $C_1$-$C_{20}$-Alkylgruppe oder -Alkylcarbonylgruppe oder eine Arylgruppe oder Alkarylgruppe mit 1 bis 20 C-Atomen im Alkylrest bedeutet oder eine der Gruppen $R^6$ bis $R^8$ Wasserstoff ist, oder 2 der 3 Gruppen $R^6$ bis $R^8$ zusammen mit Stickstoff einen gegebenenfalls durch eine, zwei oder drei $C_1$-$C_5$-Alkylgruppen substituierten heterocyclischen Ring mit 5 oder 6 Gliedern aus Kohlenstoff- sowie gegebenenfalls ein oder zwei Sauerstoff- und|oder stickstoffatomen bilden, **dadurch gekennzeichnet**, daß man ein Gemisch aus Dioxan und einem Olefin der allgemeinen formel

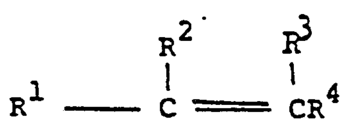

(II)

in der $R^1$, $R^2$, $R^3$ und $R^4$ die genannte Bedeutung haben, gelöst in einem inerten Lösungsmittel, bei einer Temperatur von +20°C bis –78°C mit Schwefeltrioxid,verdünnt mit Stickstoff oder einem gegen $SO_3$ inerten Gas, in einem Fallfilmreaktor bzw. Dünnschichtreaktor sulfoniert und das erhaltene 1,2-Sulton der allgemeinen Formel

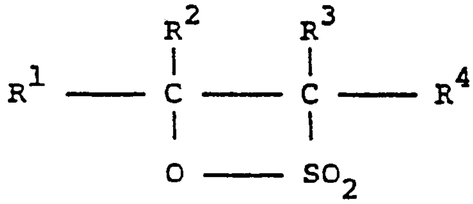

(III)

in der $R^1$ bis $R^4$ die genannte Bedeutung haben, ohne Isolierung entweder

a) durch Umsetzen mit einer Verbindung der allgemeinen Formel $R^5OH$ oder deren Alkalisalz, worin $R^5$ die genannte Bedeutung hat, zur entsprechenden Sulfonsäure der allgemeinen Formel

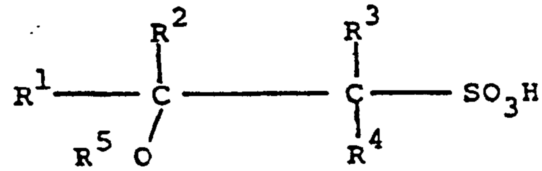 (IV)

umsetzt und diese gegebenenfalls nach Umsetzen mit der ensprechenden Base in das Alkali- oder Ammoniumsalz überführt oder

b) durch Umsetzen mit einem sekundären oder tertiären Amin der allgemeinen Formel $R^6R^7R^8N$, worin $R^6$, $R^7$ und $R^8$ die genannte Bedeutung haben, in die entsprechende Betainverbindung der allgemeinen Formel

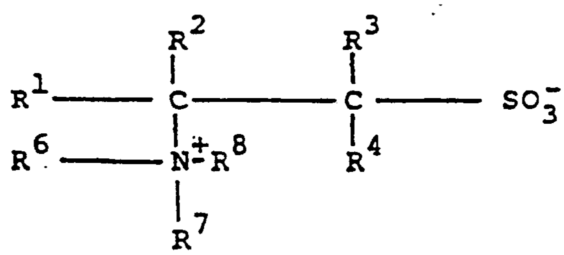 (V)

überführt.

2. Verfahren nach Anspruch 1, **dadurch gekennzeichnet**, daß man die Sulfonierung bei einer Temperatur von +5°C bis –20°C durchführt.

3. Verfahren nach den Ansprüchen 1 und 2, **dadurch gekennzeichnet**, Dass man als Lösungsmittel für das Olefin ein Alkan oder einen halogenierten Kohlenwasserstoff verwendet.

4. Verfahren nach den Ansprüchen 1 bis 3, **dadurch gekennzeichnet**, dass man die Sulfonierung im Molverhältnis Olefin : $SO_3$ = 1 : 1 bis 1 : 5 durchführt.

5. Verfahren nach Anspruch 4, **dadurch gekennzeichnet**, dass man ein Molverhältnis Olefin : $SO_3$ von 1 : 1 verwendet.

6. Verfahren nach Anspruch 1 dadurch **gekennzeichnet** dass man eine Schwefeltrioxidkonzentration im Inertgas bzw. in Luft oder Stickstoff von 1 bis 20 Vol.-% verwendet.

7. Verwendung nach dem Verfahren gemäß den Ansprüchen 1 bis 6 hergestellten Verbindungen als oberflächenaktive Mittel.

**Revendications**

1. Procédé de préparation de composés d'éthanesulfone éventuellement substitués de formule générale:

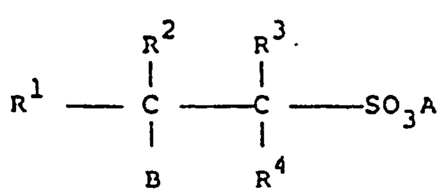 (1)

où $R^1$ représente un groupe alcoyle en $C_1$ à $C_{20}$, $R^2$, $R^3$ et $R^4$ représentent chacun un hydrogène ou un groupe alcoyle en $C_1$ à $C_{20}$, A représente un hydrogène, un métal alcalin ou l'ammonium ou une charge négative et B représente un groupe $R^5O$, où $R^5$ représente un groupe alcoyle en $C_1$ à $C_{20}$ ou alcoylcarbonyle en $C_1$ à $C_{20}$, un groupe alcénylcarbonyle en $C_2$ à $C_{20}$, un groupe alcadiène en $C_4$ à $C_{20}$ ou un groupe alcadiénylcarbonyle en $C_4$ à $C_{20}$, un groupe aryle, un groupe alcaryle dont le groupe alcoyle a de 1 à 20 atomes de carbone, ou un

groupe amidoalcoyle carboxylique dont le groupe alcoyle a de 1 à 20 atomes de carbone, ou B représente un groupe $—O(CH_2CH_2O)_nR^5$,

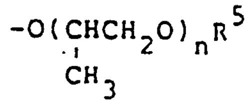

ou 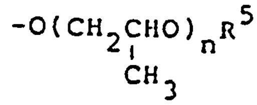

avec n = 1 à 50 ou un groupe $R^6R^7R^8N$, où $R^6$, $R^7$ et $R^8$ représentent chacun un groupe alcoyle en $C_1$ à $C_{20}$ ou un groupe alcoylcarbonyle en $C_1$ à $C_{20}$, ou un groupe aryle ou un groupe alcaryle dont le groupe alcoyle a de 1 à 20 atomes de carbone, ou l'un des groupes $R^6$ à $R^8$ est un hydrogène, ou 2 des 3 groupes $R^6$ à $R^8$ forment ensemble avec l'azote un noyau hétérocyclique éventuellement substitué par un, deux ou trois groupes alcoyle en $C_1$ à $C_5$, avec 5 ou 6 chaînons de carbone ainsi qu'éventuellement un ou deux atomes d'oxygène et/ou d'azote, caractérisé en ce qu'on sulfone un mélange de dioxanne et d'une oléfine de formule générale

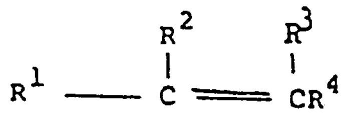

(II)

où $R^1$, $R^2$, $R^3$ et $R^4$ ont la signification mentionnée, dissous dans un solvant inerte, à une température de +20°C à –78°C avec de l'anhydride sulfurique, dilué avec de l'azote ou d'un gaz inerte vis-à-vis du $SO_3$, dans un réacteur à couche mince, et en ce qu'on transforme la 1,2-sultone obtenue de formule générale

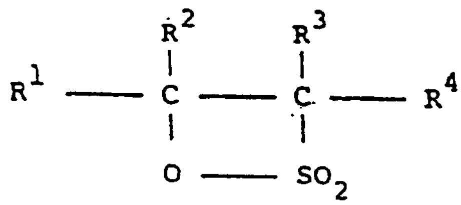

(III)

où $R^1$ à $R^4$ ont la signification mentionnée, sans l'isoler, soit

a) par réaction avec un composé de formule générale $R^5OH$ ou son sel alcalin, où $R^5$ a la signification mentionnée, pour donner l'acide sulfonique correspondant de formule générale

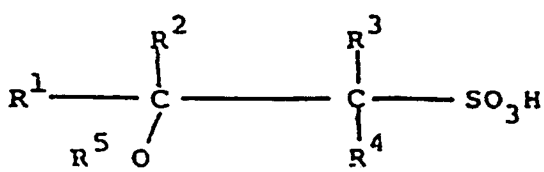

(IV)

et l'on transforme celui-ci le cas échéant après réaction avec la base correspondante en le sel de métal alcalin ou d'ammonium, soit

b) par réaction avec une amine secondaire ou tertiaire de formule générale $R^6R^7R^8N$, où $R^6$, $R^7$ et $R^8$ ont la signification mentionnée, pour donner le composé de bêtaine correspondant de formule générale

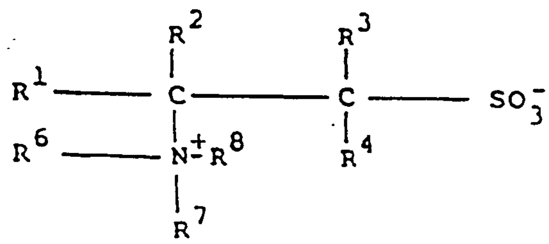 (V)

2. Procédé selon la revendication 1, caractérisé en ce qu'on conduit la sulfonation à une température allant de +5°C à −20°C.

3. Procédé selon les revendications 1 et 2, caractérisé en ce qu'on utilise comme solvant pour l'oléfine un alcane ou un hydrocarbure halogéné.

4. Procédé selon les revendications 1 à 3, caractérisé en ce qu'on conduit la sulfonation dans un rapport molaire oléfine : $SO_3$ de 1 : 1 à 1 : 5.

5. Procédé selon la revendication 4, caractérisé en ce qu'on utilise un rapport molaire oléfine : $SO_3$ de 1 : 1.

6. Procédé selon la revendication 1, caractérisé en ce qu'on utilise une concentration d'anhydride sulfurique dans le gaz inerte ou selon le cas dans l'air ou l'azote de 1 à 20% en volume.

7. Application des composés préparés selon le procédé des revendications 1 à 6 comme agents tensioactifs.

## Claims

1. Process for the preparation of optionally substituted Ethane sulphone compounds of the general formula

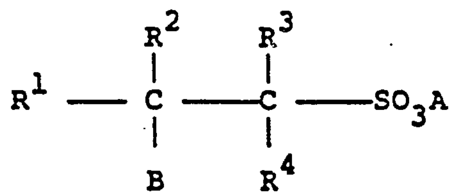 (1)

wherein $R^1$ is a $C_1$ to $C_{20}$-alkyl group, $R^2$, $R^3$ and $R^4$ are each hydrogen or a $C_1$ to $C_{20}$ alkyl group, A is hydrogen, alkali metal or ammonium or a negative charge and B is a group $R^5O$, wherein $R^5$ is a $C_1$ to $C_{20}$-alkyl or alkyl carbonyl group, $C_2$ to $C_{20}$-alkenyl carbonyl group, $C_4$ to $C_{20}$-alkadienyl group or -alkadienylcarbonyl group, aryl group, alkaryl having from 1 to 20 C-atoms in the alkyl residue, or a carboxylic amidoalkyl group having from 1 to 20 C-atoms in the alkyl group, or B is a group $—O(CH_2CH_2O)_nR^5$ or

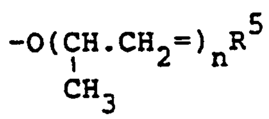 or

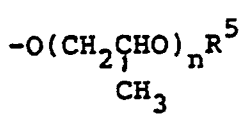,

n being from 1 to 50, or B is a group $R^6R^7R^8N$ wherein $R^6$, $R^7$ and $R^8$ are a $C_1$ to $C_{20}$-alkyl group or -alkyl carbonyl group or an aryl group or alkaryl group having from 1 to 20 C-atoms in the alkyl residue or one of the groups $R^6$ to $R^8$ is hydrogen, or 2 of the 3 groups $R^6$ to $R^8$ form, together with nitrogen,an heterocyclic ring having 5 or 6 members consisting of carbon atoms and, optinally, one or two oxygen and/or nitrogen atoms, said ring optionally being substituted by one, two or three $C_1$ to $C_5$-alkyl groups, characterized in that a mixture of dioxane and ananolefin of the general formula

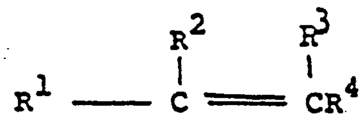

(II)

wherein $R^1$, $R^2$, $R^3$ and $R^4$ have the meaning as defined dissolved in an inert solvent is sulphonated at a temperature of +20°C to −78°C with sulphur trioxide, diluted with nitrogen or a gas being inert to $SO_3$ in a falling film and thin layer reactor, respectively, the resulting 1,2-sultone of the general formula

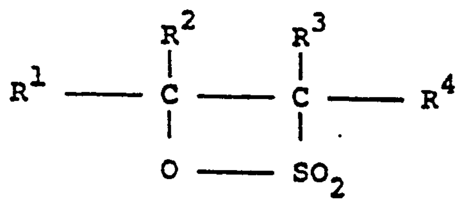

(III)

wherein $R^1$ to $R^4$ have the meaning as defined is reacted without isolation either

a) with a compound of the general formula $R^5OH$ or the alkali salt thereof wherein $R^5$ has the meaning as defined to form the corresponding sulphonic acid of the general formula

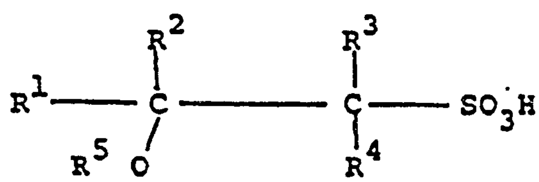

(IV)

and, this acid is transferred to the corresponding alkali or amonium salt optionally after reaction with the corresponding base or

b) with a secondary or tertiary amine of the general formula $R^6R^7R^8N$ wherein $R^6$, $R^7$ and $R^8$ have the meaning a defined, to form the corresponding betaine compound of the general formula

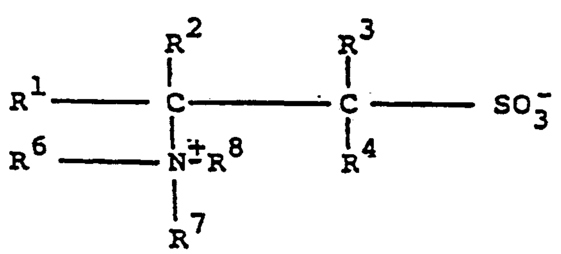

(V)

2. Process as claimed in claim 1, characterized in that the sulphonation is carried out at a temperature from +5°C to −20°C.

3. Process as claimed in claims 1 and 2, characterized in that an alkane or an halogenated hydrocarbon is used as the solvent for the olefin.

4. Process as claimed in claims 1 to 3 characterized in that the sulphonation is carried out in a molar ratio olefin : $SO_3$ = 1 : 1 to 1 : 5.

5. Process as claimed in claim 4 characterized in that a molar ratio olefin : $SO_3$ of 1 : 1 is used.

6. Process as claimed in claim 1, characterized in that a concentration of sulphur trioxide of 1 to 20% by volume in the inert gas and air or nitrogen respectively is used.

7. Use of the compounds as prepared according to a process as claimed in claims 1 to 6 as surface active compounds.

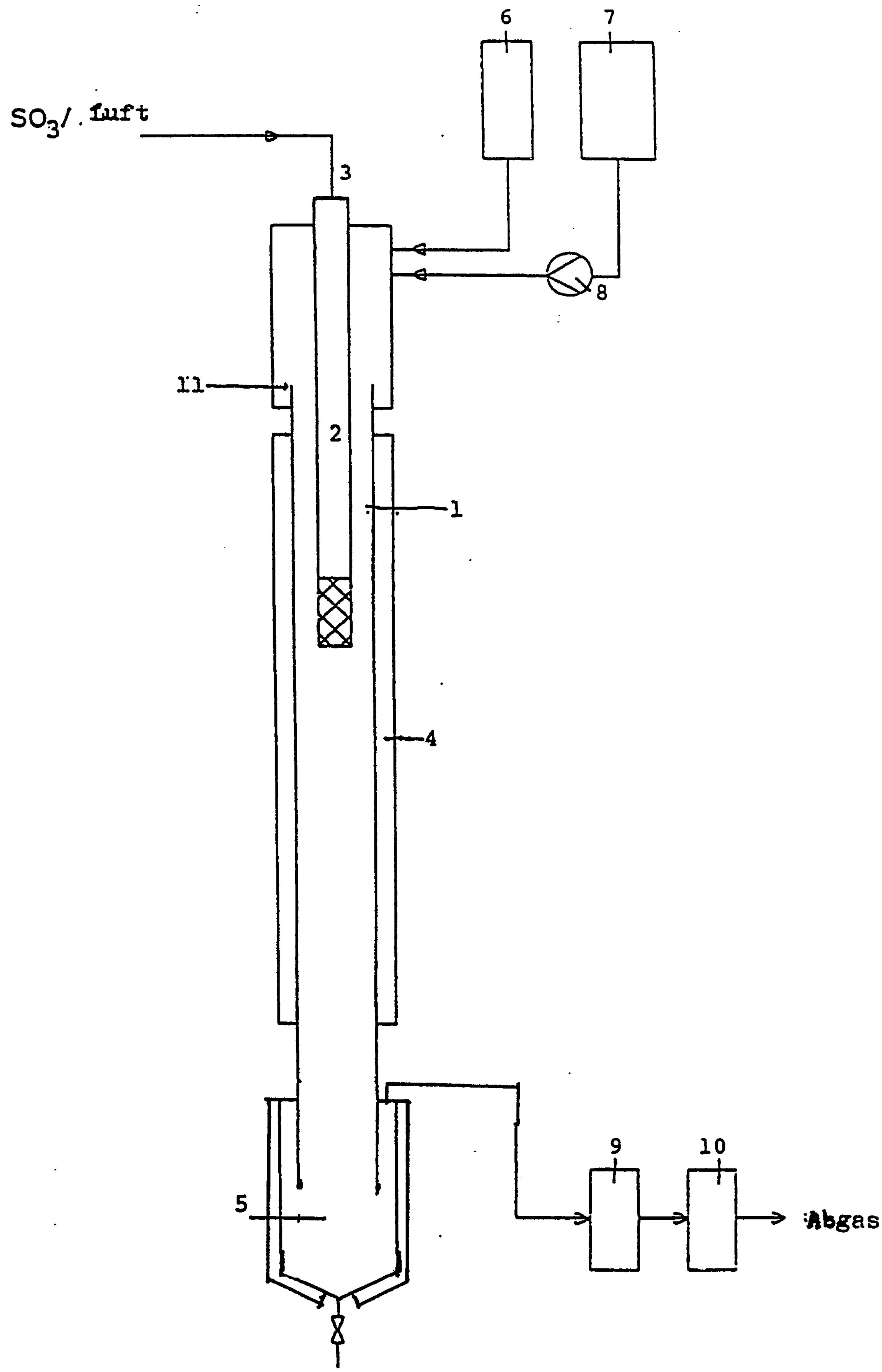
6
7
$SO_3$/ Luft
3
8
11
2
1
4
5
9
10
Abgas